Europäisches Patentamt

European Patent Office

Office européen des brevets

(19)

(11) Publication number: **0 341 674**
A2

## EUROPEAN PATENT APPLICATION

(12)

(21) Application number: **89108344.6**

(22) Date of filing: **09.05.89**

(51) Int. Cl.4: **C12N 15/00 , C12N 9/88**

(30) Priority: **09.05.88 JP 110401/88**
**15.11.88 JP 286728/88**

(43) Date of publication of application:
**15.11.89 Bulletin 89/46**

(84) Designated Contracting States:
**AT BE CH DE ES FR GB IT LI NL SE**

(71) Applicant: **MITSUI TOATSU CHEMICALS, Inc.**
**2-5 Kasumigaseki 3-chome**
**Chiyoda-Ku Tokyo 100(JP)**

(72) Inventor: **Ishiwata, Kenich**
**314, Kamikurata-cho Totsuka-ku**
**Yokohama-shi Kanagawa 244(JP)**
Inventor: **Yoshino, Setsuo**
**2882, Iijima-cho Sakae-ku**
**Yokohama-shi Kanagawa 244(JP)**
Inventor: **Iwamori, Satoru**
**2070, Iijima-cho Sakae-ku**
**Yokohama-shi Kanagawa 244(JP)**
Inventor: **Suzuki, Tadashi**
**1-33-4-102, Maruyama Isogo-ku**
**Yokohama-shi Kanagawa 235(JP)**
Inventor: **Makiguchi, Nobuyoshi**
**2-12-23, Honkugenuma**
**Fujisawa-shi Kanagawa 251(JP)**

(74) Representative: **Kinzebach, Werner, Dr. et al**
**Patentanwälte Reitstötter, Kinzebach und**
**Partner Sternwartstrasse 4 Postfach 86 06 49**
**D-8000 München 86(DE)**

(54) Cloned tryptophan synthase gene and recombinant plasmid containing the same.

(57) Diclosed is a cloned gene encoding tryptophan synthase A and/or B. A recombinant plasmid containing the tryptophan synthase gene, an E. coli transformant transformed with the recombinant plasmid and a process of producing tryptophan synthase by culturing the transformant are also disclosed.

EP 0 341 674 A2

**Cloned Tryptophan Synthase Gene and Recombinant Plasmid Containing the Same**

## BACKGROUND OF THE INVENTION

I. Field of the Invention

This invention relates to a cloned tryptophan synthase gene, a recombinant plasmid containing the same, an E. coli transformant transformed with the recombinant plasmid, and to a process of producing tryptophan synthase.

II. Description of the Related Art

Tryptophan synthase is a so called multifunctional enzyme and one of its functions is to catalyze the reaction between indole and L-serine to produce L-tryptophan (Miles, E.W. (1979) Adv. Enzymol., Vol. 49, p. 127-186).

Heretofore, tryptophan synthase genes originating from Escherichia coli (Hershfield, V. et al., (1974) Proc. Natl. Acad. Sci. U.S.A., Vol. 71, p. 3455-3459), Bacillus subtilis (Henner, D.J. et al., (1984), Gene, vol. 34, pp.169-177), Brevibacterium lactofermentum, (Matsui, K. et al., (1986), Agric. Biol. Chem. vol. 51, pp.823-828), Salmonella typhimurium (Kawasaki, H. et al., (1987) J. Biol. Chem. vol. 262, pp. 10678-10683) and Saccharomyces cerevisiae (Waltz, A. et al., (1978), Proc. Natl. Acad. Sci. U.S.A., vol. 75, pp.6172-6176) have been cloned.

However, the tryptophan synthase produced by utilizing such a cloned gene originating from the above-mentioned mesophiles has unsatisfactory thermostability.

In general, in the industrial production of a product utilizing an enzyme, it is preferred to employ a thermostable enzyme because the reaction can be conducted at a higher temperature, the rate of consumption of the enzyme is low and the contamination with other mesophilic bacteria can be prevented. Thus, if a thermostable tryptophan synthase can be produced in a large amount utilizing the genetic engineering technique, it is advantageous for the industrial production of, for example, tryptophan by the reaction between indole and L-serine.

## SUMMARY OF THE INVENTION

Accordingly, the object of the present invention is to provide a cloned thermostable tryptophan synthase.

Another object is to provide a recombinant plasmid containing the thermostable tryptophan synthase gene, which is capable of producing the thermostable tryptophan synthase in a host cell.

Still another object of the present invention is to provide a transformant transformed with the recombinant plasmid of the present invention which produces the thermostable tryptophan synthase.

Still another object of the present invention is to provide a process of producing the thermostable tryptophan synthase by utilizing a genetic engineering technique.

The present inventors succeeded in cloning a thermostable tryptophan synthase gene originating from a moderate thermophile, Bacillus stearothemophilus and in constructing a recombinant plasmid containing the thermostable tryptophan synthase gene, as well as expressing the gene in E. coli to complete the present invention.

That is, the present invention provides a cloned gene comprising a region encoding tryptophan synthase B with the amino acid sequence of:

```
MERVPNEHGR  FGDFGGKFVP  ETLMLPLEEI  EAELDKALAD

ESFKQEYIRI  LQHYSGRPTP  LTFAPNLTRQ  LGGAKMYLKR

EDLNHTGAHK  INNAIGQALL  AKRMGKKKLI  AETGAGQHGV

AAATVAAHFG  MDCIVFMGEE  DIKRQELNVF  RMKLLGAEVV

PVSSGNRTLK  DATNEAIRYW  VAHCDDHFYM  IGSVVGPHPY

PKMVREFQRI  IGDEAKEQFL  ACEGKLPDVI  VACVGGGSNA

IGMFYPFLQD  DVRLVGVEAA  GKGIDTPYHA  ATITKGTKGV

IHGAMTYLLQ  DEYGQIVEPY  SISAGLDYPG  VGPEHAYLAS

IGRVRYESVT  DEEAVAAFRL  LAQTEGIIPA  IESAHAVAKA

VELAQSMSPD  ETVLICLSGR  GDKDVQTMMR  HLGAKEGEDV

AAIR
```

(wherein A represents alanine, C represents cystein, D represents aspargic acid, E represents glutamic acid, F represents phenylalanine, G represents glycine, H represents histidine, I represents isoleucine, K represents lysine, L represents leucine, M represents methionine, N represents asparagine, P represents proline, Q represents glutamine, R represents alginine, S represents serine, T represents threonine, V represents valine, W represents tryptophan and Y represents tyrosine).

The present invention also provides a cloned gene comprising a region encoding tryptophan synthase A with the amino acid sequence of:

```
MLLLSVNPPL  FIPFIVAGDP  SPEVTVDLAL  ALEEAGADLL

ELGVPYSDPL  ADGPTIQRAA  ARALAGNMTL  PKAIHLVAEM

RKKGVTIPII  LFTYYNPVLQ  LGEESFFALA  RENGANGVLI

PDLPFEESGP  LRELGERFDL  PLISLVAPTS  KQRIERIASV

AQGFLYCVSS  LGVTGMRETL  PESLGDFVSE  VKRHSRVPVA

VGFGISTPEQ  VAMLKEVCDG  VVIGSALVQK  VEQLGERLLA

PEEKEAAIAE  FAAYARSLAA  PLHAPCSLR
```

(wherein the letters represent the same meaning as mentioned above).

The present invention also provides a recombinant plasmid comprising a gene containing a region encoding tryptophan synthase B and/or A with the above-described amino acid sequence, which is capable of expressing tryptophan synthase B and/or A in a host cell.

The present invention still further provides an E. coli transformant transformed with the recombinant plasmid of the present invention, which transformant is capable of producing the thermostable tryptophan synthase.

The present invention still further provides a process of producing tryptophan synthase comprising the steps of culturing the E. coli transformant of the present invention in a condition to produce the tryptophan synthase, and recovering the produced tryptophan synthase.

By the present invention, a thermostable tryptophan synthase gene was first cloned and a recombinant plasmid containing the thermostable tryptophan synthase gene was first provided. Thus, by the present

3

invention, production of a thermostable tryptophan synthase by the genetic engineering technique was first accomplished. In other words, the mass production of the thermostable tryptophan synthase by the genetic engineering technique was first attained by the present invention. Thus, the present invention is advantageous for the production of, for example, tryptophan from indole and L-serine in a commercial scale.

## BRIEF DESCRIPTION OF THE DRAWINGS

Fig. 1 is a restriction map of the recombinant plasmid pISY10 of the present invention;

Fig. 2 is a restriction map of the recombinant plasmid pISY21 of the present invention;

Fig. 3 is a restriction map of a DNA fragment with a size of about 4.6 kb originating from the chromosomal DNA of Bacillus stearothermophilus, which is inserted in the recombinant plasmid pISY21 of the present invention, which restriction map also shows the approximate position of the trpA gene and trpB gene;

Fig. 4 is a restriction map of the recombinant plasmid pISY73 of the present invention;

Fig. 5 shows the scheme employed in the dideoxy method for determining the base sequence of a DNA fragment inserted in the plasmid pISY73 of the present invention;

Fig. 6 shows the scheme employed in the Maxam-Gilbert's method for determining the base sequence of a DNA fragment inserted in the plasmid pISY73 of the present invention;

Fig. 7 shows the base sequence of the Hind III-Eco RV fragment with a size of about 2.5 kb, which is inserted in the recombinant plasmid pISY73 of the present invention;

Fig. 8 shows the amino acid sequence of the thermostable tryptophan synthase B encoded by the cloned gene of the present invention; and

Fig. 9 shows the amino acid sequence of the thermostable tryptophan synthase A encoded by the cloned gene of the present invention.

## DETAILED DESCRIPTION OF THE PREFERRED EMBODIMENTS

The gene of the present invention encodes thermostable tryptophan synthase A and/or tryptophan synthase B, of which amino acid sequences are described above. The gene exists, for example, in the chromosomal DNA of a moderate thermophile, Bacillus stearothermophilus. Examples of the Bacillus stearothermophilus strains include those deposited in Institute for Fermentation, Osaka (IFO), Japan under the accession number of IFO 12983 and IFO 13737. The gene may also be derived from other sources and may be synthesized.

The cloning of the gene may be accomplished, for example, as follows:

First of all, a B. stearothermophilus strain having a chromosomal DNA containing the thermostable tryptophan synthase gene is cultured by the conventional method, and the chromosomal DNA is extracted and purified from the cells by the conventional method such as phenol method (Saito, H. et al., (1963), Biochim. Biophys. Acta, vol. 72, pp.619-629).

Then the chromosomal DNA is digested with one or more of appropriate restriction enzymes. For the digestion of the chromosomal DNA of B. stearothermophilus, various restriction enzymes such as Hpa II, Acc I, Sau3A I, Hpa I and Sca I may be employed. The degree of digestion can be controlled by controlling the amount of the enzyme used and/or the reaction time.

The digested chromosomal DNA is then recombined with a vector in the presence of a DNA ligase by the conventional method (see, for example, Maniatis, T. et al., (1982), Molecular Cloning, Cold Spring Harbor Lab.) to form recombinant DNAs. The vector may preferably be a plasmid vector which can replicate in a host cell, preferably in E. coli. Examples of the plasmid vectors which can replicate in E. coli include commercially available ColE1 series vectors such as pBR322 and pUC19. Since the size of the DNA fragments containing the thermostable tryptophan synthase gene, as well as the restriction enzymes used for cutting out the fragments were discovered by the present inventors as described in the Examples hereinbelow described, only the chromosomal DNA fragments having the desired size may be ligated with the vector after selecting the desired DNA fragments by the conventional method such as agarose gel electrophoresis. As the DNA ligase, those originating from T4 phage may preferably be used.

The thus prepared recombinant DNAs are then used for transforming a host such as E. coli mutants which lacks the ability to produce tryptophan synthase and which requires tryptophan. Examples of the E.

coli mutants lacking the ability to produce tryptophan synthase, which requires tryptophan include E. coli K12 W3110 MT-10347 which was deposited in accordance with Budapest Treaty in Fermentation Research Institute (FRI), Ibaraki, Japan on March 15, 1988, under the accession number FERM BP-2361. The transformation may be accomplished by the conventional method such as calcium chloride method (Mandel, O. et al., (1970), J. Mol. Biol. vol. 53, pp.159-162).

Then the transformants which lost the auxotroph to tryptophan are selected. This selection can be carried out by culturing the transformants in a culture medium not containing tryptophan. The fact that the transformant lost the auxotroph to tryptophan implies that the cell was transformed with a recombinant plasmid containing the gene encoding tryptophan synthase which can be expressed in the cell and that tryptophan synthase was produced thereby.

The recombinant plasmid contained in the transformants which lost the auxotroph to tryptophan can be extracted from the transformant to provide the recombinant plasmid of the present invention. The extraction of the recombinant plasmid from the transformant may be conducted by the conventional method such as described in Brinboim, H. et al., (1979), Nucleic Acids Res., vol. 7, pp. 1513-1523. The fact that the recombinant plasmid contains the tryptophan synthase gene can be confirmed by cutting out the DNA fragment which was recombined with the vector in the previous step using the restriction enzymes used for digesting the chromosomal DNA, and by determining the base sequence of the cut out DNA fragment. In the Examples hereinbelow described, two recombinant plasmids pISY10 and pISY21 were obtained.

To further promote the efficiency of the expression of the tryptophan synthase gene, it is desired to recombine the DNA fragment containing the tryptophan synthase gene with an expression vector having a strong promoter such as trp promoter, lacUV5 promoter, tac promoter and $\lambda P_L$ promoter which are known in the art, such that the DNA fragment is inserted downstream the strong promoter. Such an expression vector having a strong promoter and a cloning site downstream thereof is known in the art and examples thereof include pKK223-3 commercially available from Pharmacia. In the Examples described later, a recombinant plasmid pISY73 was prepared by this method. Needless to say, since the recombinant plasmid of the present invention can express the tryptophan synthase gene in the host cell, in addition to the promoter sequence, the plasmid has at least a replication origin which enables the plasmid to replicate in the cell, and in cases where the host cell is prokaryotic, an SD sequence upstream the tryptophan synthase gene. The recombinant plasmid may preferably further comprise a terminater sequence for stopping the transcription and an antibiotic resistant gene which is useful as a marker or useful for preventing contamination with other microorganisms. Expression vectors containig these sequences are known in the art and commercially available. The above-mentioned pKK223-3 vector is an example.

It should be noted, however, the method of cloning the gene is not restricted to the method described above. For example, the DNA fragment containing the thermostable tryptophan synthase gene may be prepared by recovering messenger RNAs in a cell producing the enzyme and by preparing cDNAs from the messenger RNAs by using reverse transcriptase. The DNA fragments obtained by this method can be processed in the same manner as described above.

By transforming E. coli with the recombinant plasmid of the present invention, the E. coli transformant of the present invention which is capable of producing the thermostable tryptophan synthase may be obtained. The transformation of E. coli can be accomplished as described above.

The present invention further provides a process of producing tryptophan synthase by culturing the E. coli transformant of the present invention in a condition to produce the tryptophan synthase and recovering the produced tryptophan synthase. The culture of the E. coli transformant can be carried out by the conventional method for culturing E. coli. That is, the E. coli transformant may be cultured in a conventional medium containing assimilable carbon source and nitrogen source, as well as inorganic ions and possibly amino acids and/or vitamines under aerobic condition at a temperature of preferably about 37°C. When the promoter of the recombinant plasmid contained in the transformant is one controlled by isopropyl-$\beta$-thiogalactoside (IPTG), such as tac promoter, this compound may be added to the medium. Further, when the recombinant plasmid has an antibiotic gene, the antibiotic may be added to the medium so as to prevent the contamination by other microorganisms. Still further, to promote the production of the tryptophan synthase, a compound such as indole acrylic acid which promotes the production of the tryptophan synthase may be added to the medium, if desired.

The thermostable tryptophan synthase may be purified from the culture by the conventional method well-known in the art after disrupting the cells by ultrasonication or the like. By heat-treating the disrupted cells, the thermostable tryptophan synthase can be recovered efficiently. It should be noted, however, in addition to the purified enzyme, the culture medium per se in which the transformant was cultured, as well as the crude extract of the enzyme, whole transformant cells and derivatives thereof may also be used as the enzyme source in a commercial plant for producing the thermostable tryptophan synthase.

The invention will now be described in more detail by way of examples thereof. It should be noted, however, the examples are presented for illustration purpose only and should not be interpreted in any restrictive way.

Example 1

(a) Isolation of Chromosomal DNA of Bacillus stearothermophilus

Bacillus stearothermophilus (deposited in IFO, Osaka, Japan under an accession number of IFO 13737) was inoculated to 2 liters of L medium (10 g/l of bactotryptone, 5 g/l of yeast extract and 5 g/l of sodium chloride, pH 7.2) and was cultured under shaking at 55°C for 15 hours, followed by being collected by centrifugation. The cells were suspended in 160 ml of 0.15 M NaCl-50 mM EDTA (pH 8.0) solution. To this suspension, 160 mg of lysozyme was added and the resulting mixture was gently stirred at 37°C for 20 minutes.

Then 4 ml of 20 wt% of SDS solution was added to the mixture and the resulting mixture was left to stand at 65°C for 20 minutes. Then 8 mg of proteinase K (commercially available from Boehringer Mannheim) was added thereto and the resultant was left to stand at 37°C for 1 hour.

To the resulting mixture, 160 ml of phenol saturated with 0.15 M NaCl-50 mM EDTA (pH 8.0) was added and the resultant was gently stirred. This mixture was centrifuged (10,000 rpm, 15 minutes) and the upper aqueous phase was recovered.

To the thus obtained solution, twice volume of cold ethanol was added and fibrous precipitate was collected by winding the same about a glass rod. The precipitate was successively immersed in 70 wt%, 80 wt% and 90 wt% of ethanol for several minutes each time and then dried. The obtained product was dissolved in 40 ml of 0.1M NaCl-0.15M sodium citrate solution (pH 7.0).

To the resulting crude DNA solution, were added 200 μl of 6 mg/l of ribonuclease A (commercially available from Boehringer Mannheim) and 200 μl of 1000 U/ml of ribonuclease T1 (commercially available from Boehringer Mannheim), and resulting mixture was stirred for 1.5 hours at 37°C.

To this solution, 40 ml of phenol saturated with 0.15 M NaCl-50 mM EDTA (pH 8.0) was added and the resulting mixture was gently stirred. Then the mixture was centrifuged (10,000 rpm, 15 mininutes) and the upper aqueous phase was recovered.

To the resulting mixture, was added twice volume of ethanol and the fibrous precipitate was recovered by winding the same about a glass rod. The precipitate was successively immersed in 70 wt%, 80 wt% and 90 wt% of ethanol for several minutes each time and then dried. The obtained product was then dissolved in 20 ml of 10 mM Tris-HCl (pH 8.0) - 1 mM EDTA solution (hereinafter referred to as "TE buffer").

The thus obtained DNA solution was dialyzed against 2 liters of TE bufffer to obtain 20 ml of TE buffer containing 4.5 mg of chromosomal DNA.

(b) Preparation of Chromosomal DNA Fragments

To 1350 μl of aliquote of the TE buffer obtained in (a) which contained 300 μg of DNA, 30 units of restriction enzyme Hpa II (commercially available from Boehringer Mannheim) was added. The reaction was conducted in 1500 μl of a reaction medium containing 10 mM Tris-HCl (pH 7.5), 7 mM MgCl₂ and 7 mM 2-mercaptoethanol. The reaction mixture was left to stand at 37°C for 1 hour to partially cut the chromosomal DNA.

To this solution, equivolume of phenol/chloroform (phenol: chloroform = 1:1) saturated with TE buffer was added and the resulting mixture was gently stirred. Then the mixture was centrifuged (15,000 rpm, 5 minutes) and the upper aqueous phasee was collected.

Twice volume of cold ethanol was added to the collected solution and the generated precipitate was dissolved in 300 μl of TE buffer after drying. This DNA solution was subjected to 10 - 40 wt% sucrose density gradient centrifugation (20°C, 26,000 rpm, 24 hours) and two fractions of about 4 - 6 kb and about 6 - 10 kb were recovered. After dialyzing the each fraction against TE buffer, DNA was collected by ethanol precipitation and the obtained DNA was dissolved in TE buffer.

(c) Ligation of Chromosomal DNA Fragments with Plasmid Vector

Forty micrograms of a conventional vector plasmid pUC19 (manufactured by Takara Shuzo Co., Ltd., Kyoto, Japan) was completely digested with a restriction enzyme Acc I (commercially available from Boehringer Mannheim), and the digested mass was treated with alkaline phosphatase. Twenty micrograms each of the thus treated plasmid vectors was mixed with each of the fraction of the chromosomal DNA fragments prepared in (b), respectively, and each of the mixtures was allowed to react separately in the presence of 200 units of T4 DNA ligase in 4 ml of a reaction medium containing 5 mM MgCl₂, 10 mM dithiothreitol, 1 mM ATP, 66 mM Tris-HCl buffer (pH 7.5) at 16°C for 16 hours.

This solution was used as it is in the next step of transforming E. coli.

(d) Cloning of Tryptophan synthase Gene

Transformation of E. coli was conducted using the reaction mixture prepared in (c) containing the recombinant plasmid as follows: E. coli K12 W3110 MT-10347 (deposited in Fermentation Research Institute (FRI), Ibaraki, Japan on March 15, 1988 under an accession number FERM BP-2361) which is a mutant lacking the production ability of tryptophan synthase and requiring tryptophan was inoculated to 50 ml of L medium (10 g/l of bactotryptone, 5 g/l of yeast extract and 5 g/l of sodium chloride, pH 7.2) and was cultured under shaking at 37°C for 3 hours. The cultured cells were then collected by centrifugation.

The cells were suspended in 2 ml of 0.1 M CaCl₂ and the obtained suspension was left to stand at 0°C for 30 minutes. Thereafter, the suspension was centrifuged and the collected cells were re-suspended in 40 ml of 0.1 M CaCl₂.

The thus prepared cell suspension was poured into two test tubes in the amount of 20 ml each. To each of the cell suspensions, the reaction mixture prepared in (c) was added and the each of the resulting mixtures was left to stand at 0°C for 3 hours and then at 42°C for 2 minutes.

The cells were collected by centrifugation and 10 ml of L medium was added to the cells. The cells were cultured at 37°C for 30 minutes and then collected by centrifugation.

After suspending the cells in 10 ml of 0.85 wt% NaCl, the cells were collected by centrifugation and then re-suspended in 1 ml of 0.85 wt% NaCl solution.

Each of the thus prepared cell suspensions was applied to Vogel and Bonner's medium (0.2 g/l of MgSO₄・7H₂O, 2 g/l of citric acid・H₂O, 10 g/l of K₂HPO₄, 3.5 g/l of NaNH₄PO₄・4H₂O and 4 g/l of glucose) to which 100 mg/l of ampcillin, 25 mg/l of isopropyl-β-thiogalactoside (IPTG), 10 g/l of Casamino acid and 15 g/l of agar were added, and cultured thereon at 37°C for 2 days.

On the agar plate medium on which E. coli transformed with the recombinant plasmid prepared from the fraction of the chromosomal DNA with a size of 6 - 10 kb was cultured, 22 colonies were formed. The E. coli collected from a representative colony was named as Escherichia coli MT-10471 and was deposited in FRI on March 15, 1988, under an accession number of FERM BP-2362.

On the other hand, on the agar plate medium on which E. coli transformed with the recombinant plasmid prepared from the fraction of the chromosomal DNA with a size of 4 - 6 kb was cultured, 22 colonies were formed. The E. coli collected from a representative colony was named as Escherichia coli MT-10472 and was deposited in FRI on March 15, 1988, under an accession number of FERM BP-2363.

(e) Isolation of Recombinant Plasmid Contained in E. coli.

Recombinant plasmids were isolated from E. coli MT-10471 and E. coli MT-10472 as follows:

E. coli MT-10471 or E. coli MT-10472 was inoculated to 50 ml of L medium and was cultured under shaking at 37°C for 15 hours. The cells were then collected by centrifugation.

The cells were suspended in 2 ml of 50 mM glucose-25 mM Tris-HCl-10 mM EDTA (pH 8.0) containing 2 mg of lysozyme.

To this suspension, 4 ml of 0.2 N NaOH solution containing 1 wt% SDS was added and the resulting mixture was stirred. Then 3 ml of 3 M CH₃COONa (pH 5.2) solution was added thereto and the resultant was left to stand at 4°C for 5 minutes. Then the resulting mixture was centrifuged and the supernatant was collected.

To the thus obtained solution, equivolume of phenol/chloroform (phenol:chloroform = 1:1) was added and the resulting mixture was gently stirred. The resultant was centrifuged (10,000 rpm, 5 minutes) and the supernatant was collected.

To the thus collected upper aqueous phase, twice volume of cold ethanol was added and the generated precipitate was dissolved in 1 ml of TE buffer after drying.

To the resulting DNA solution, 20 µl of 1 mg/l of ribonuclease A (commercially available from Boehringer Mannheim) was added and the resultant was left to stand at 37°C for 20 minutes.

To this solution, equivolume of phenol/chloroform (phenol:chloroform = 1:1) was added and the mixture was gently stirred. This mixture was then centrifuged (10,000 rpm, 5 minutes) and the upper aqueous phase was collected.

The thus obtained DNA solution was purified by column chromatography on Bio-Gel A-50m column (commercially available from Bio-Rad), and the plasmid DNA was collected by ethanol precipitation.

By the process described above, about 50 µg of plasmid DNA was recovered from each of E. coli MT-10471 and E. coli MT-10472, and each plasmid DNA was dissolved in 100 µl of TE buffer.

In the analysis of the plasmid DNA hereinbelow described, the thus obtained plasmid DNAs were used.

(f) Analysis of Recombinant Plasmid

The recombinant plasmid isolated from E. coli MT-10471 was named as pISY10. This plasmid has a size of about 12.2 kb and contains about 9.5 kb DNA insert which can be cut out with Eco RI and Hind III.

The recombinant plasmid isolated from E. coli MT-10472 was named as pISY21. This plasmid has a size of about 7.3 kb and contains about 4.6 kb DNA insert which can be cut out with Eco RI and Hind III.

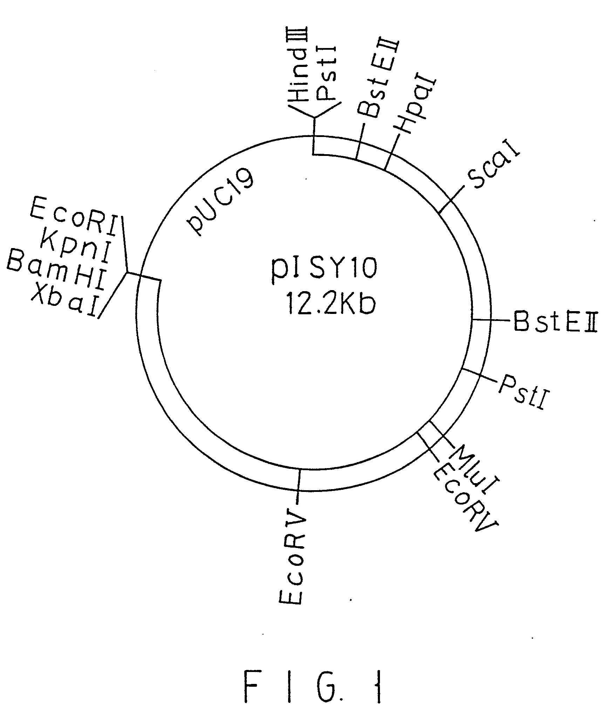

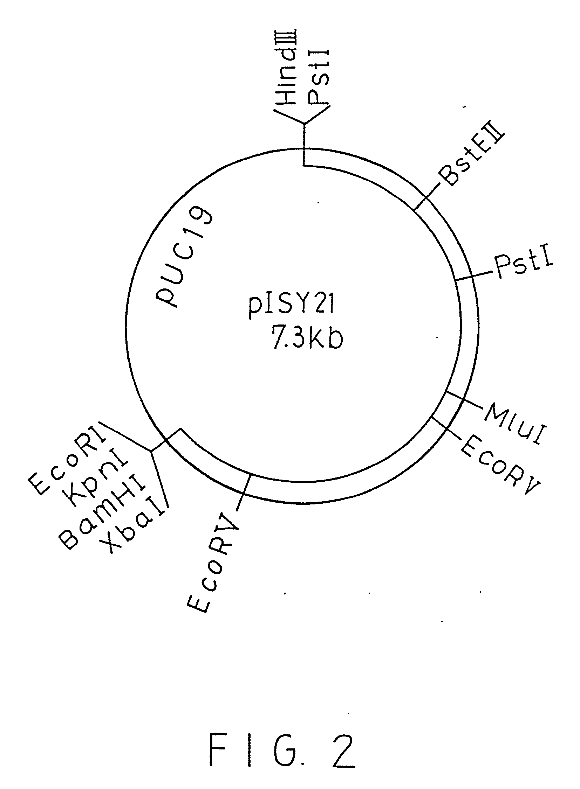

The restriction maps of pISY10 and pISY21 are shown in Figs. 1 and 2, respectively. From Figs. 1 and 2, it can be seen that the DNA fragment inserted in pISY21 is a part of the DNA fragment inserted in pISY10.

The fact that the DNA fragments inserted in pISY10 or pISY21 originate from Bacillus stearothermophilus was confirmed by Southern blotting method as follows:

The Southern blotting method was conducted in accordance with the conventional method (T. Maniatis et al (1982), Molecular Cloning, Cold Spring Harbor Lab).

The chromosomal DNA of Bacillus stearothermophilus IFO 13737 was separated in the same manner as in (a).

On the other hand, recombinant plasmid pISY10 and pISY21 were prepared as described in (e).

The chromosomal DNA was cut with Eco RI and was subjected to agarose electrophoresis, followed by being transferred to a nitrocellulose filter paper.

The recombinant plasmid pISY10 and pISY21 were digested with Eco RI and Hind III and only the inserted DNA fragments were collected and labelled with $^{32}$P.

Using the DNA labelled with $^{32}$P as a probe, the hybridization of the probe and the chromosomal DNA on the nitrocellulose filter paper was conducted.

Autoradiography revealed that the chromosomal DNA had a region which hybridized with either probe originating from pISY10 or pISY21.

By these results, it was confirmed that either of the DNA fragment inserted in pISY10 or in pISY21 was originated from the chromosomal DNA.

(g) Confirmation of the Reproducibility of Transformation

E. coli K12 W3110 MT-10347 lacking the ability to produce tryptophan synthase and requiring tryptophan was transformed in the same manner as described in (d) with the recombinant plasmid pISY10 or pISY21 isolated in (e), and was applied to a L medium agar plate containing 100 mg/l of ampicillin. From the generated colonies, 20 colonies were picked up and the auxotrophy to tryptophan was checked. As a result, in all of the checked colonies, the auxotroph to tryptophan was lost. By this, it was confirmed that the pISY10 and pISY21 had a DNA carrying the tryptophan synthase gene.

(h) Subcloning and Analysis of DNA Fragments

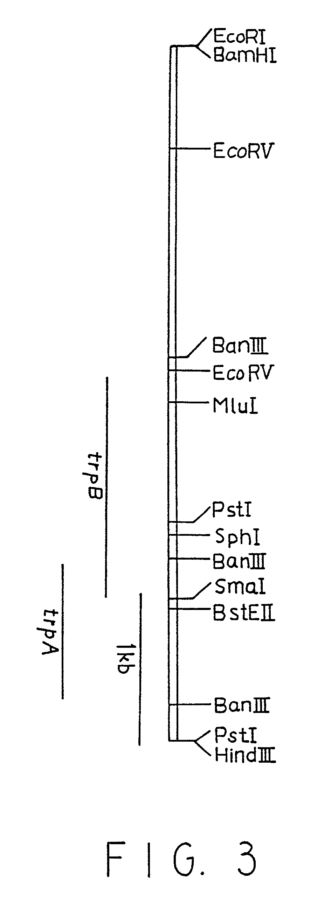

The DNA fragment originating from the chromosomal DNA of Bacillus stearothermophilus, which was inserted in pISY21 had a size of about 4.6 kb. The detailed restriction map thereof is shown in Fig. 3. From the DNA fragment inserted in pISY21, various DNA fragments were prepared, which lacked a portion of the DNA insert in pISY21. Each of the various fragments were inserted in the polylinker region downstream the tac promoter of a commercially available expression vector pKK223-3 (commercially available from Pharmacia). With each of these recombinant plasmids, E. coli MT-10347 (trpAB⁻), E. coli ATCC-23717

8

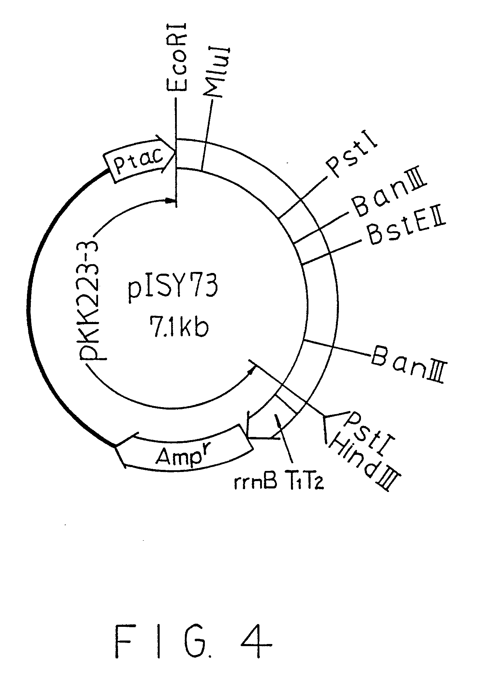

(trpA⁻) and E. coli ATCC-23718 (trpB⁻) were transformed. By checking whether the auxotroph to tryptophan of these transformants is lost or not, approximate positions of the trpA gene and trpB gene were determined. Among the DNA inserts originating from pSIY21, the fragment in which the trpA gene and the trpB gene are considered to be contained is the Eco RV-Hind III fragment with a size of about 2.5 kb. The recombinant plasmid pISY73 was the plasmid prepared by inserting this fragment in the polylinker region of pKK223-3 cut with Sma I-Hind III. The restriction map of pISY73 is shown in Fig. 4. E. coli was transformed with pISY73 to obtain a transformant E. coli MT-10474 which was deposited in FRI on September 9, 1988 under an accession number of FERM BP-2364.

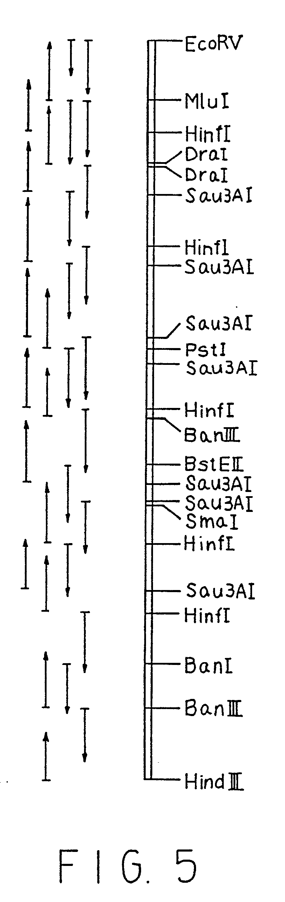

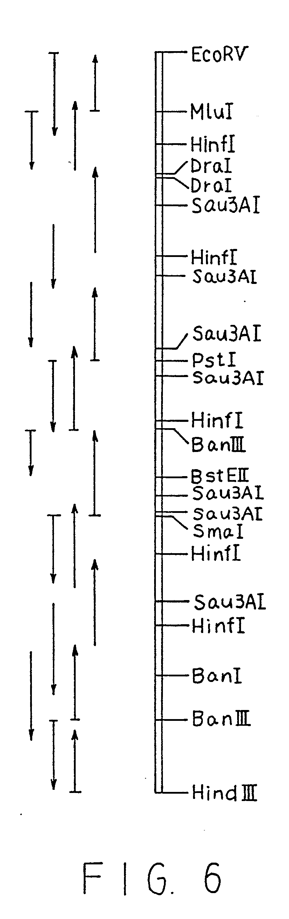

The DNA sequence of the Eco RV-Hind III insert of pISY73, which had a size of about 2.5 kb was determined by the conventional dideoxy method (Messing, J. (1983), Methods in Enzymology, vol. 101, p. 20 - 78) according to the scheme shown in Fig. 5 using an M13 series vector (Messing, J. (1983) Methods in Enzymology, vol. 101, pp.20-78) and by the conventional Maxam-Gilbert method (Maxam, A.M. and Gilbert, W. (1980), Methods in Enzymology, Vol. 65, p. 499-560) according to the scheme shown in Fig. 6.

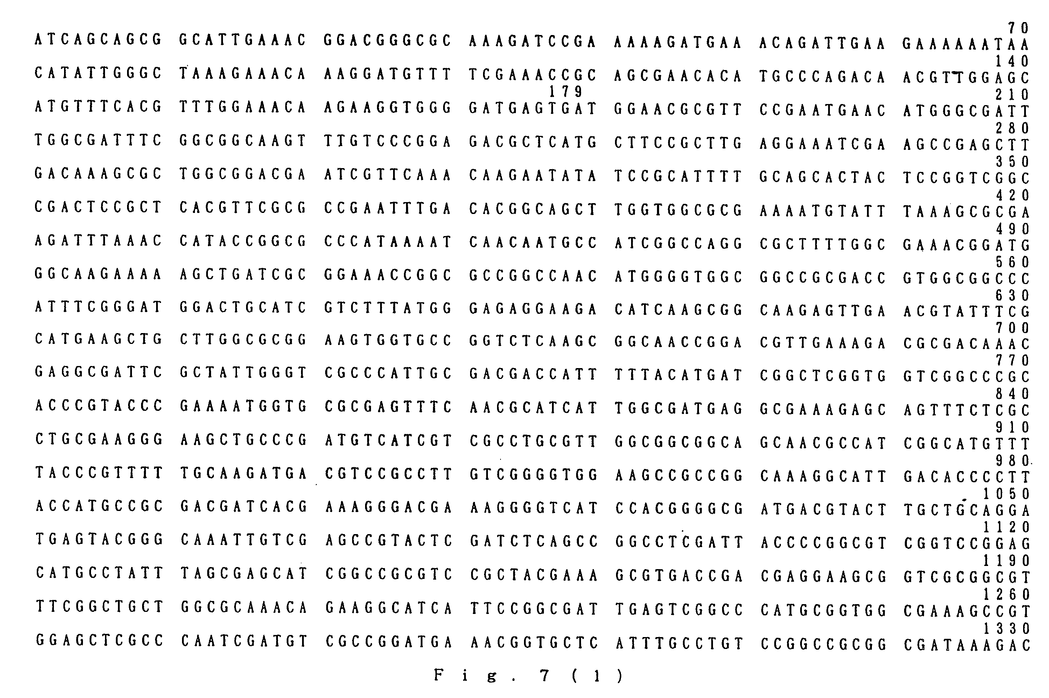

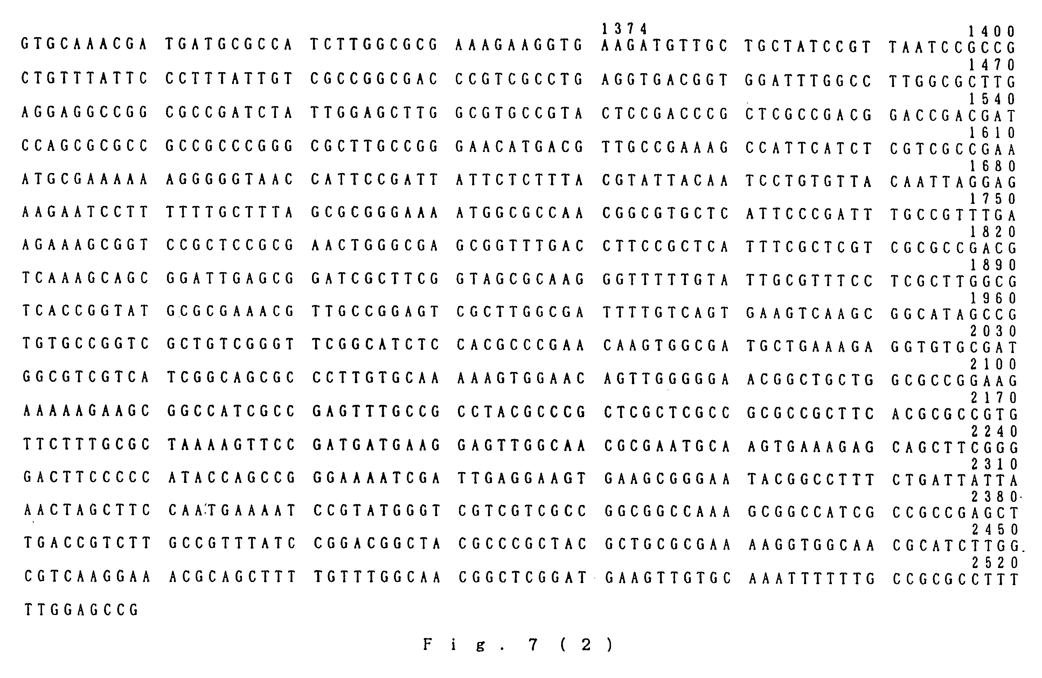

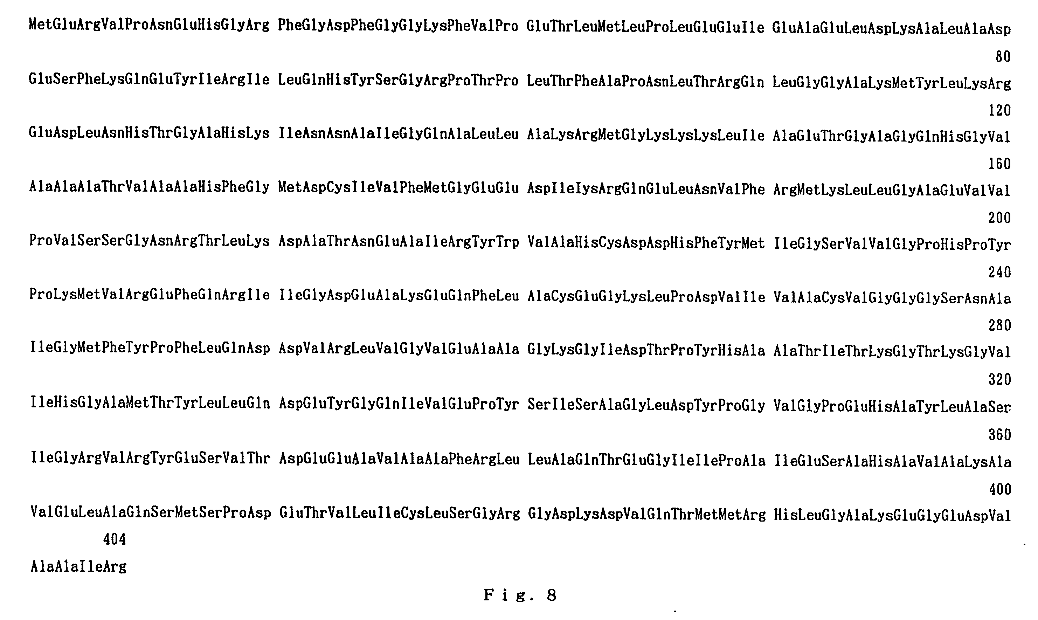

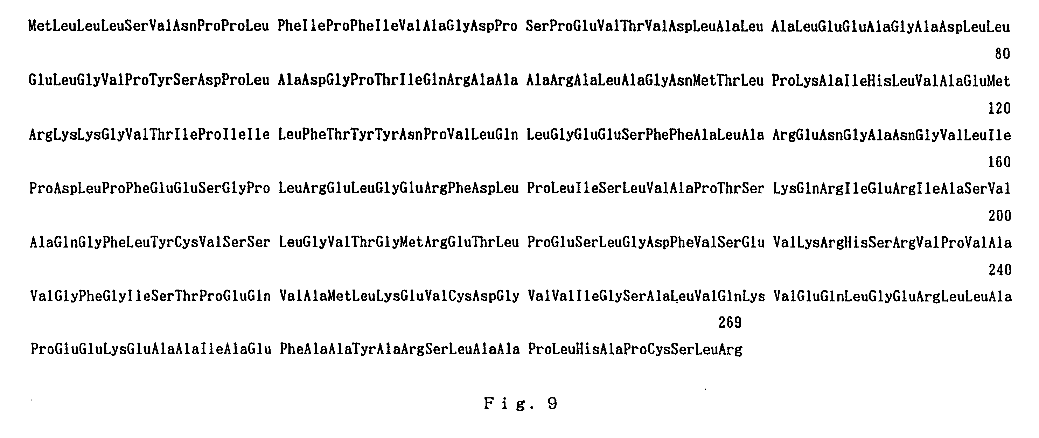

As a result, it was confirmed that the DNA insert had a base sequence shown in Fig. 7. In this base sequence, the existence of two open reading frames starting from 179th base and 1374th base, which corresponds to the trpB gene and trpA gene, respectively, was confirmed. The two open reading frames co-owned 17 base pairs. The amino acid sequence of trpB is shown in Fig. 8, and that of trpA is shown in Fig. 9.

(i) Production of Tryptophan Synthase

E. coli MT-10471, E. coli MT-10472 and E. coli MT-10474 were separately inoculated to 50 ml of Vogel and Bonner's medium containing 100 mg/l of ampicillin, 25 mg/l of IPTG and 10 g/l of Casamino acid, and were cultured under shaking at 37°C for 24 hours. Thereafter, the cells were collected by centrifugation.

After washing the collected cells, the cells were suspended in 10 ml of 100 mM Tris-HCl buffer (pH 7.8) containing 0.1 mM pyridoxal. By ultrasonication, an extract which did not contain cells was prepared and the trptophan synthase activity of the extract was determined by the Yanofsky's method (Yanofsky, C. et al., (1962), Methods in Enzymology, Vol. 5, p. 794-806).

For comparison, E. coli K12 W3110 MT-10347 which is the host of the transformants prepared as described above was cultured in the same medium (but containing 20 mg/l of tryptophan) and the tryptophan synthase activity was determined in the same manner as described above.

The trptophan synthase activity of each strain is shown in the table below. One unit of tryptophan synthase activity is defined as the amount of the enzyme, which yields 0.1 μmol of tryptophan in the reaction at 37°C for 20 minutes.

Table

| Strain | Enzyme Activity (Units/mg protein) |
|---|---|
| MT-10471 | 2 |
| MT-10472 | 12 |
| MT-10474 | 69 |
| MT-10347 | 0 |

The extract of MT-10474 which did not contain the cells was heat-treated at 65°C for 10 minutes and then the tryptophan synthase activity was determined. As a result, about 90% of the enzyme activity was remained.

Although the invention was described based on the preferred embodiments thereof, it is apparent for those skilled in the art that various modifications may be made without departing from the spirit and scope of the present invention.

**Claims**

1. A cloned gene comprising a region encoding tryptophan synthase B with the amino acid sequence of:

```
MERVPNEHGR FGDFGGKFVP ETLMLPLEEI EAELDKALAD

ESFKQEYIRI LQHYSGRPTP LTFAPNLTRQ LGGAKMYLKR

EDLNHTGAHK INNAIGQALL AKRMGKKKLI AETGAGQHGV

AAATVAAHFG MDCIVFMGEE DIKRQELNVF RMKLLGAEVV

PVSSGNRTLK DATNEAIRYW VAHCDDHFYM IGSVVGPHPY

PKMVREFQRI IGDEAKEQFL ACEGKLPDVI VACVGGGSNA

IGMFYPFLQD DVRLVGVEAA GKGIDTPYHA ATITKGTKGV

IHGAMTYLLQ DEYGQIVEPY SISAGLDYPG VGPEHAYLAS

IGRVRYESVT DEEAVAAFRL LAQTEGIIPA IESAHAVAKA

VELAQSMSPD ETVLICLSGR GDKDVQTMMR HLGAKEGEDV

AAIR
```

(wherein A represents alanine, C represents cystein, D represents aspargic acid, E represents glutamic acid, F represents phenylalanine, G represents glycine, H represents histidine, I represents isoleucine, K represents lysine, L represents leucine, M represents methionine, N represents asparagine, P represents proline, Q represents glutamine, R represents alginine, S represents serine, T represents threonine, V represents valine, W represents tryptophan and Y represents tyrosine).

2. A cloned gene comprising a region encoding tryptophan synthase A with the amino acid sequence of:

```
MLLLSVNPPL FIPFIVAGDP SPEVTVDLAL ALEEAGADLL

ELGVPYSDPL ADGPTIQRAA ARALAGNMTL PKAIHLVAEM

RKKGVTIPII LFTYYNPVLQ LGEESFFALA RENGANGVLI

PDLPFEESGP LRELGERFDL PLISLVAPTS KQRIERIASV

AQGFLYCVSS LGVTGMRETL PESLGDFVSE VKRHSRVPVA

VGFGISTPEQ VAMLKEVCDG VVIGSALVQK VEQLGERLLA

PEEKEAAIAE FAAYARSLAA PLHAPCSLR
```

(wherein the letters represent the same meaning as in claim 1).

3. The cloned gene of claim 1 or 2 which has a DNA sequence of:

ATGGAACGCG TTCCGAATGA ACATGGGCGA TTTGGCGATT

TCGGCGGCAA GTTTGTCCCG GAGACGCTCA TGCTTCCGCT

TGAGGAAATC GAAGCCGAGC TTGACAAAGC GCTGGCGGAC

GAATCGTTCA AACAAGAATA TATCCGCATT TTGCAGCACT

ACTCCGGTCG GCCGACTCCG CTCACGTTCG CGCCGAATTT

GACACGGCAG CTTGGTGGCG CGAAAATGTA TTTAAAGCGC

GAAGATTTAA ACCATACCGG CGCCCATAAA ATCAACAATG

CCATCGGCCA GGCGCTTTTG GCGAAACGGA TGGGCAAGAA

AAAGCTGATC GCGGAAACCG GCGCCGGCCA ACATGGGGTG

GCGGCCGCGA CCGTGGCGGC CCATTTCGGG ATGGACTGCA

TCGTCTTTAT GGGAGAGGAA GACATCAAGC GGCAAGAGTT

GAACGTATTT CGCATGAAGC TGCTTGGCGC GGAAGTGGTG

CCGGTCTCAA GCGGCAACCG GACGTTGAAA GACGCGACAA

ACGAGGCGAT TCGCTATTGG GTCGCCCATT GCGACGACCA

TTTTTACATG ATCGGCTCGG TGGTCGGCCC GCACCCGTAC

CCGAAAATGG TGCGCGAGTT TCAACGCATC ATTGGCGATG

AGGCGAAAGA GCAGTTTCTC GCCTGCGAAG GGAAGCTGCC

11

```
CGATGTCATC GTCGCCTGCG TTGGCGGCGG CAGCAACGCC

ATCGGCATGT TTTACCCGTT TTTGCAAGAT GACGTCCGCC

TTGTCGGGGT GGAAGCCGCC GGCAAAGGCA TTGACACCCC

TTACCATGCC GCGACGATCA CGAAAGGGAC GAAAGGGGTC

ATCCACGGGG CGATGACGTA CTTGCTGCAG GATGAGTACG

GGCAAATTGT CGAGCCGTAC TCGATCTCAG CCGGCCTCGA

TTACCCCGGC GTCGGTCCGG AGCATGCCTA TTTAGCGAGC

ATCGGCCGCG TCCGCTACGA AAGCGTGACC GACGAGGAAG

CGGTCGCGGC GTTTCGGCTG CTGGCGCAAA CAGAAGGCAT

CATTCCGGCG ATTGAGTCGG CCCATGCGGT GGCGAAAGCC

GTGGAGCTCG CCCAATCGAT GTCGCCGGAT GAAACGGTGC

TCATTTGCCT GTCCGGCCGC GGCGATAAAG ACGTGCAAAC

GATGATGCGC CATCTTGGCG CGAAAGAAGG TGAAGATGTT

GCTGCTATCC GTTAATCCGC CGCTGTTTAT TCCCTTTATT

GTCGCCGGCG ACCCGTCGCC TGAGGTGACG GTGGATTTGG

CCTTGGCGCT TGAGGAGGCC GGCGCCGATC TATTGGAGCT

TGGCGTGCCG TACTCCGACC CGCTCGCCGA CGGACCGACG

ATCCAGCGCG CCGCCGCCCG GGCGCTTGCC GGGAACATGA

CGTTGCCGAA AGCCATTCAT CTCGTCGCCG AAATGCGAAA

AAAGGGGGTA ACCATTCCGA TTATTCTCTT TACGTATTAC

AATCCTGTGT TACAATTAGG AGAAGAATCC TTTTTTGCTT

TAGCGCGGGA AAATGGCGCC AACGGCGTGC TCATTCCCGA

TTTGCCGTTT GAAGAAAGCG GTCCGCTCCG CGAACTGGGC

GAGCGGTTTG ACCTTCCGCT CATTTCGCTC GTCGCGCCGA

CGTCAAAGCA GCGGATTGAG CGGATCGCTT CGGTAGCGCA

AGGGTTTTTG TATTGCGTTT CCTCGCTTGG CGTCACCGGT

ATGCGCGAAA CGTTGCCGGA GTCGCTTGGC GATTTTGTCA
```

12

```
GTGAAGTCAA  GCGGCATAGC  CGTGTGCCGG  TCGCTGTCGG

GTTCGGCATC  TCCACGCCCG  AACAAGTGGC  GATGCTGAAA

GAGGTGTGCG  ATGGCGTCGT  CATCGGCAGC  GCCCTTGTGC

AAAAAGTGGA  ACAGTTGGGG  GAACGGCTGC  TGGCGCCGGA

AGAAAAAGAA  GCGGCCATCG  CCGAGTTTGC  CGCCTACGCC

CGCTCGCTCG  CCGCGCCGCT  TCACGCGCCG  TGTTCTTTGC

GC
```

4. The cloned gene of claim 1 or 2, originating from Bacillus stearothermophilus.

5. A DNA fragment having the base sequence shown in Fig. 7.

6. A recombinant plasmid comprising a gene containing a region encoding tryptophan synthase B with the amino acid sequence set forth in claim 1, which is capable of expressing tryptophan synthase B in a host cell.

7. A recombinant plasmid comprising a gene containing a region encoding tryptophan synthase A with the amino acid sequence set forth in claim 2, which is capable of expressing tryptophan synthase A in a host cell.

8. The recombinant plasmid of claim 6 or 7, wherein the gene has a DNA sequence set forth in claim 3.

9. A recombinant plasmid comprising the DNA fragment of claim 3, which is capable of expressing tryptophan synthase B gene and tryptophan synthase A gene.

10. A recombinant plasmid pISY10;
pISY21 and
pISY73.

11. Esherichia coli transformed with the recombinant plasmid of claims 6; 7; 8 or 9.

12. Escherichia coli MT-10471 FERM BP-2362;
Escherichia coli MT-10472 FERM BP-2363 and Escherichia coli MT-10474 FERM BP-2364.

13. A process of producing tryptophan synthase comprising the steps of culturing an Escherichia coli of claim 11 transformed with the recombinant plasmids of claims 6, 8 or 9 or culturing an E. coli of claim 12 in a condition to produce the tryptophan synthase, and recovering the produced tryptophan synthase.

14. A process of producing tryptophan synthase comprising the steps of culturing the Escherichia coli of claim 11 transformed with the recombinant plasmid of claim 7 in a condition to produce the tryptophan synthase, and recovering the produced tryptophan synthase.

F I G. 1

EP 0 341 674 A2

F I G. 2

F I G. 3

F I G. 4

F I G. 5

F I G. 6

```
ATCAGCAGCG  GCATTGAAAC  GGACGGGCGC  AAAGATCCGA  AAAAGATGAA  ACAGATTGAA  GAAAAAATAA
CATATTGGGC  TAAAGAAACA  AAGGATGTTT  TCGAAACCGC  AGCGAACACA  TGCCCAGACA  ACGTTGGAGC
ATGTTTCACG  TTTGGAAACA  AGAAGGTGGG  GATGAGTGAT  GGAACGCGTT  CCGAATGAAC  ATGGGCGATT
TGGCGATTTC  GGCGGCAAGT  TTGTCCCGGA  GACGCTCATG  CTTCCGCTTG  AGGAAATCGA  AGCCGAGCTT
GACAAAGCGC  TGGCGGACGA  ATCGTTCAAA  CAAGAATATA  TCCGCATTTT  GCAGCACTAC  TCCGGTCGGC
CGACTCCGCT  CACGTTCGCG  CCGAATTTGA  CACGGCAGCT  TGGTGGCGCG  AAAATGTATT  TAAAGCGCGA
AGATTTAAAC  CATACCGGCG  CCCATAAAAT  CAACAATGCC  ATCGGCCAGG  CGCTTTTGGC  GAAACGGATG
GGCAAGAAAA  AGCTGATCGC  GGAAACCGGC  GCCGGCCAAC  ATGGGGTGGC  GGCCGCGACC  GTGGCGGCCC
ATTTCGGGAT  GGACTGCATC  GTCTTTATGG  GAGAGGAAGA  CATCAAGCGG  CAAGAGTTGA  ACGTATTTCG
CATGAAGCTG  CTTGGCGCGG  AAGTGGTGCC  GGTCTCAAGC  GGCAACCGGA  CGTTGAAAGA  CGCGACAAAC
GAGGCGATTC  GCTATTGGGT  CGCCCATTGC  GACGACCATT  TTTACATGAT  CGGCTCGGTG  GTCGGCCCGC
ACCCGTACCC  GAAAATGGTG  CGCGAGTTTC  AACGCATCAT  TGGCGATGAG  GCGAAAGAGC  AGTTTCTCGC
CTGCGAAGGG  AAGCTGCCCG  ATGTCATCGT  CGCCTGCGTT  GGCGGCGGCA  GCAACGCCAT  CGGCATGTTT
TACCGTTTTT  TGCAAGATGA  CGTCCGCCTT  GTCGGGGTGG  AAGCCGCCGG  CAAAGGCATT  GACACCCCTT
ACCATGCCGC  GACGATCACG  AAAGGGACGA  AAGGGGTCAT  CCACGGGGCG  ATGACGTACT  TGCTGCAGGA
TGAGTACGGG  CAAATTGTCG  AGCCGTACTC  GATCTCAGCC  GGCCTCGATT  ACCCCGGCGT  CGGTCCGGAG
CATGCCTATT  TAGCGAGCAT  CGGCCGCGTC  CGCTACGAAA  GCGTGACCGA  CGAGGAAGCG  GTCGCGGCGT
TTCGGCTGCT  GGCGCAAACA  GAAGGCATCA  TTCCGGCGAT  TGAGTCGGCC  CATGCGGTGG  CGAAAGCCGT
GGAGCTCGCC  CAATCGATGT  CGCCGGATGA  AACGGTGCTC  ATTTGCCTGT  CCGGCCGCGG  CGATAAAGAC
```

Fig. 7 (1)

```
1374
     GTGCAAACGA TGATGCGCCA TCTTGGCGCG AAAGAAGGTG AAGATGTTGC TGCTATCCGT TAATCCGCCG   1400
     CTGTTTATTC CCTTTATTGT CGCCGGCGAC CCGTCGCCTG AGGTGACGGT GGATTTGGCC TTGGCGCCTG   1470
     AGGAGGCCCG CGCCGATCTA TTGGAGCTTG GCGTGCCGTA CTCCGACCCG CTCGCCGACG GACCGACGAT   1540
     CCAGCGCGCC GCCGCGCGGG CGCTTGCCGG GAACATGACG TTGCCGAAAG CCATTCATCT CGTCGCCGAA   1610
     ATGCGAAAAA AGGGGGTAAC CATTCCGATT ATTCTCTTTA CGTATTACAA TCCTGTGTTA CAATTAGGAG   1680
     AAGAATCCTT TTTTGCTTTA GCGCGGGAAA ATGGCGCCAA CGGCCGTGCTC ATTCCCGATT TGCCGTTTGA   1750
     AGAAAGCGGT CCGCTCCGCG AACTGGGCCGA GCGGTTTGAC CTTCCGCTCA TTTCGCTCGT CGCGCCGACG   1820
     TCAAAGCAGC GGATTGAGCG GATCGCTTCG GTAGCGCAAG GGTTTTTGTA TTGCGTTTCC TCGCTTGGCG   1890
     TCACCGGTAT GCCGCGAAACG TTGCCCGGAGT CGCTTGGCGA TTTGTCAGT GAAGTCAAGA GGCATAGCCG   1960
     TGTGCCGGTC GCTGTCGGGT TCGGCATCTC CACGCCCCGAA CAAGTGGCGA TGCTGAAAGA GGTGTGCGAT   2030
     GGCGTCGTCA TCGGCAGCGC CCTTGTGCAA AAAGTGGAAC AGTTGGGGGA ACGGCTGCTG GCGCCGGAAG   2100
     AAAAGAAGC GGCCATCGCC GAGTTTGCCCG CCTACCGCCCG CTCGCTCGCC GCGCCGGCTTC ACGGCCGCTG   2170
     TTCTTTGCGC TAAAAGTTCC GATGATGAAG GAGTTGGCAA CGCCGAATGCA AGTGAAAGAG CAGCTTCGGG   2240
     GACTTCCCCC ATACCAGCCG GGAAAATCGA TTGAGGAAGT GAAGCGGGAA TACGGCCCTTT CTGATTATTA   2310
     AACTAGCTTC CAATGAAAAT CCGTATGGGT CCGTCGTCGGT GCCGGCCAAA GCGGCCAGCT CCCGGCAGCT   2380
     TGACCGTCTT GCCGTTTATC CGGACGGGCTA CGCCCCGCTAC CGCCCCGCTAC AAGGTGGCAA CGCATCTTGG   2450
     CGTCAAGGAA ACGCAGCTTT TGTTTGGCAA CGGCTCGGAT GAAGTTGTGC AAGGTGGCAA CCGCGCCTTT   2520
     TTGGAGCCG
```

Fig. 7 (2)

MetGluArgValProAsnGluHisGlyArg PheGlyAspPheGlyGlyLysPheValPro GluThrLeuMetLeuProLeuGluGluIle GluAlaGluLeuAspLysAlaLeuAlaAsp

GluSerPheLysGlnGluTyrIleArgIle LeuGlnHisTyrSerGlyArgProThrPro LeuThrPheAlaProAsnLeuThrArgGln LeuGlyGlyAlaLysMetTyrLeuLysArg

GluAspLeuAsnHisThrGlyAlaHisLys IleAsnAsnAlaIleGlyGlnAlaLeuLeu AlaLysArgMetGlyLysLysLysLeuIle AlaGluThrGlyAlaGlyGlnHisGlyVal

AlaAlaAlaThrValAlaAlaHisPheGly MetAspCysIleValPheMetGlyGluGlu AspIleIysArgGlnGluLeuAsnValPhe ArgMetLysLeuLeuGlyAlaGluValVal

ProValSerSerGlyAsnArgThrLeuLys AspAlaThrAsnGluAlaIleArgTyrTrp ValAlaHisCysAspAspHisPheTyrMet IleGlySerValValGlyProHisProTyr

ProLysMetValArgGluPheGlnArgIle IleGlyAspGluAlaLysGluGlnPheLeu AlaCysGluGlyLysLeuProAspValIle ValAlaCysValGlyGlyGlySerAsnAla

IleGlyMetPheTyrProPheLeuGlnAsp AspValArgLeuValGlyValGluAlaAla GlyLysGlyIleAspThrProTyrHisAla AlaThrIleThrLysGlyThrLysGlyVal

IleHisGlyAlaMetThrTyrLeuLeuGln AspGluTyrGlyGlnIleValGluProTyr SerIleSerAlaGlyLeuAspTyrProGly ValGlyProGluHisAlaTyrLeuAlaSer

IleGlyArgValArgTyrGluSerValThr AspGluGluAlaValAlaAlaPheArgLeu LeuAlaGlnThrGluGlyIleIleProAla IleGluSerAlaHisAlaValAlaLysAla

ValGluLeuAlaGlnSerMetSerProAsp GluThrValLeuIleCysLeuSerGlyArg GlyAspLysAspValGlnThrMetMetArg HisLeuGlyAlaLysGluGlyGluAspVal

AlaAlaIleArg

Fig. 8

EP 0 341 674 A2

MetLeuLeuLeuSerValAsnProProLeu PheIleProPheIleValAlaGlyAspPro SerProGluValThrValAspLeuAlaLeu AlaLeuGluGluAlaGlyAlaAspLeuLeu

GluLeuGlyValProTyrSerAspProLeu AlaAspGlyProThrIleGlnArgAlaAla AlaArgAlaLeuAlaGlyAsnMetThrLeu ProLysAlaIleHisLeuValAlaGluMet

ArgLysLysGlyValThrIleProIleIle LeuPheThrTyrTyrAsnProValLeuGln LeuGlyGluGluSerPhePheAlaLeuAla ArgGluAsnGlyAlaAsnGlyValLeuIle

ProAspLeuProPheGluGluSerGlyPro LeuArgGluLeuGlyGluArgPheAspLeu ProLeuIleSerLeuValAlaProThrSer LysGlnArgIleGluArgIleAlaSerVal

AlaGlnGlyPheLeuTyrCysValSerSer LeuGlyValThrGlyMetArgGluThrLeu ProGluSerLeuGlyAspPheValSerGlu ValLysArgHisSerArgValProValAla

ValGlyPheGlyIleSerThrProGluGln ValAlaMetLeuLysGluValCysAspGly ValValIleGlySerAlaLeuValGlnLys ValGluGlnLeuGlyGluArgLeuLeuAla

ProGluGluLysGluAlaAlaIleAlaGlu PheAlaAlaTyrAlaArgSerLeuAlaAla ProLeuHisAlaProCysSerLeuArg

Fig. 9

EP 0 341 674 A2